# EUROPÄISCHE PATENTSCHRIFT

(11) **EP 4 238 502 B1**
(45) Veröffentlichungstag und Bekanntmachung des Hinweises auf die Patenterteilung: **24.04.2024**
(21) Anmeldenummer: 22159740.4
(22) Anmeldetag: 02.03.2022
(51) Int. Cl.: A61B 6/03, A61B 6/00

(54) **MEDIZINISCHE BILDGEBUNGSVORRICHTUNG MIT EINER FUNKTIONELLEN GEHÄUSESCHALE**
MEDICAL IMAGING DEVICE WITH A FUNCTIONAL HOUSING SHELL
DISPOSITIF D'IMAGERIE MÉDICALE DOTÉ D'UN COQUE DE BOÎTIER FONCTIONNEL

(43) Veröffentlichungstag der Anmeldung: 06.09.2023
(73) Patentinhaber: Siemens Healthineers AG, 91301 Forchheim (DE)
(72) Erfinder: OTT, Michael, 92676 Speinshart (DE); SEIFERT, Martin, 95447 Bayreuth (DE); WEISBROD, Erik, 81369 München (DE); STEGMEIER, Stefan, 81825 München (DE)
(74) Vertreter: Siemens Healthineers Patent Attorneys

(56) Entgegenhaltungen:
- EP-A1- 3 461 407
- CN-A- 112 043 844
- CN-U- 214 231 354
- US-A1- 2012 195 410

## Beschreibung

Die vorliegende Erfindung betrifft eine medizinische Bildgebungsvorrichtung mit einer Scannereinheit, einer Patientenlagerungsvorrichtung und einer Gehäuseeinheit, die an der Scannereinheit und/oder an der Patientenlagerungsvorrichtung angeordnet ist, wobei die Gehäuseeinheit zumindest eine funktionelle Gehäuseschale umfasst. Des Weiteren geht die Erfindung aus von einem Verfahren zu einem Reinigen einer Oberfläche einer funktionellen Gehäuseschale einer medizinischen Bildgebungsvorrichtung.

Während einer medizinischen Bildgebungsuntersuchung, wie beispielsweise einer Magnetresonanzuntersuchung, einer Computertomographie-Untersuchung (CT-Untersuchung) usw., und/oder einer Vorbereitung einer medizinischen Bildgebungsuntersuchung kann es zu einer Vielzahl an Berührungen und/oder Kontakten zwischen einer Person, insbesondere dem Patienten und/oder dem die medizinischen Bildgebungsuntersuchung betreuenden medizinischen Bedienpersonal, und einer Oberfläche der medizinischen Bildgebungsvorrichtung kommen. Zudem kann es während einer medizinischen Bildgebungsuntersuchung zu weiteren Verunreinigungen von Oberflächen der medizinischen Bildgebungsvorrichtung kommen, wie beispielweise einem Anhusten einer Oberfläche usw. Nach der medizinischen Bildgebungsuntersuchung müssen diese von dem Patienten und/oder dem medizinischen Bedienpersonal kontaminierten und/oder verunreinigten Oberflächen gereinigt werden bevor erneut eine medizinische Bildgebungsuntersuchung an einem weiteren Patienten durchgeführt werden kann. Bisher muss ein medizinisches Bedienpersonal und/oder ein Reinigungspersonal nach einer medizinischen Bildgebungsuntersuchung selbst entscheiden, welche der Oberflächen der medizinischen Bildgebungsvorrichtung einer Reinigung unterzogen werden sollen und wie eine derartige Reinigung erfolgen soll.

Aus der CN214231354U U ist eine medizinische Bildgebungsvorrichtung mit einer Scannereinheit bekannt, bei der in der Gehäuseschale ein Sensorelement zur Erfassung eines Hygienestatus vorgesehen ist, und außerdem ein Reinigungselement zur Reinigung der Oberfläche der Gehäuseschale vorhanden ist. EP3461407 A1 offenbart ein Verfahren zum Reinigen einer Gehäuseoberfläche einer medizinischen Bildgebungsanlage, bei der in der Gehäuseoberfläche Sensorelemente und Leuchtelemente vorgesehen sind. Die Leuchtelemente zeigen optisch an, wenn eine Krafteinwirkung an einem zugeordneten Sensorelement registriert wurde und damit ein Reinigungsprozess des entsprechenden lokalen Teilbereichs erfolgt ist.

Der vorliegenden Erfindung liegt insbesondere die Aufgabe zugrunde, eine Reinigung einer Oberfläche einer medizinischen Bildgebungsvorrichtung zu unterstützen. Die Aufgabe wird durch die Merkmale der unabhängigen Ansprüche gelöst. Vorteilhafte Ausgestaltungen sind in den Unteransprüchen beschrieben.

Die Erfindung geht aus von medizinischen Bildgebungsvorrichtung mit einer Scannereinheit, einer Patientenlagerungsvorrichtung und einer Gehäuseeinheit, die an der Scannereinheit und/oder an der Patientenlagerungsvorrichtung angeordnet ist, wobei die Gehäuseeinheit zumindest eine funktionelle Gehäuseschale umfasst. Erfindungsgemäß umfasst die zumindest eine funktionelle Gehäuseschale zumindest ein Sensorelement zur Erfassung eines Hygienestatus an der zumindest einen funktionellen Gehäuseschale, zumindest ein Ausgabeelement, das zu einer Ausgabe einer visuellen Anzeige des Hygienestatus ausgebildet ist, und zumindest ein Reinigungselement zur Reinigung einer Oberfläche der zumindest einen funktionellen Gehäuseschale.

Die medizinische Bildgebungsvorrichtung kann dabei beispielsweise eine Magnetresonanzvorrichtung und/oder eine Computertomographie-Vorrichtung und/oder eine PET-Vorrichtung (Positron-Emissions-Tomographie-Vorrichtung) und/oder weitere, dem Fachmann als sinnvoll erscheinende medizinische Bildgebungsvorrichtungen umfassen. Demzufolge kann eine medizinische Bildgebungsuntersuchung auch eine Magnetresonanzuntersuchung und/oder eine Computertomographie-Untersuchung und/oder eine PET-Untersuchung und/oder weitere, dem Fachmann als sinnvoll erscheinende medizinische Bildgebungsuntersuchungen umfassen. Zudem kann die medizinische Bildgebungsvorrichtung auch Strahlentherapievorrichtungen umfassen.

Die medizinische Bildgebungsvorrichtung umfasst bevorzugt eine Detektoreinheit und/oder eine Scannereinheit, die zu einer Erfassung von medizinischen Bilddaten während der medizinischen Bildgebungsuntersuchung an dem Patienten ausgebildet und/oder ausgelegt ist. Ist beispielsweise die medizinische Bildgebungsvorrichtung als Magnetresonanzvorrichtung ausgebildet, kann die Scannereinheit einen Grundmagneten zur Erzeugung eines homogenen Magnetfelds, ein Gradienten-System für eine Ortskodierung der erfassten Magnetresonanzdaten und eine Hochfrequenzantenneneinheit umfassen. Die Hochfrequenzantenneneinheit kann dabei eine fest innerhalb der Scannereinheit angeordnete Hochfrequenzantenne umfassen zu einem Aussenden eines Anregungspulses. Des Weiteren kann die Hochfrequenzantenneneinheit auch eine lokale Hochfrequenzantenne umfassen, die zur Erfassung von Magnetresonanzsignalen um den zu untersuchenden Bereich des Patienten angeordnet wird.

Des Weiteren weist die medizinische Bildgebungsvorrichtung eine Patientenlagerungsvorrichtung mit einem bewegbaren Patiententisch auf. Für eine medizinische Bildgebungsuntersuchung, insbesondere während einer Vorbereitung der medizinischen Bildgebungsuntersuchung, wird zunächst der Patient auf dem bewegbaren Patiententisch der Patientenlagerungsvorrichtung positioniert. Zudem werden für die anstehende medizinische Bildgebungsuntersuchung erforderliche Zubehöreinheiten der medizinischen Bildgebungsvorrichtung am Patienten angebracht und/oder angeordnet. Eine derartige Zubehöreinheit kann beispielsweise eine EKG-Einheit und/oder eine Infusionseinheit und/oder weitere, dem Fachmann als sinnvoll erscheinende Einheiten umfassen. Zudem kann die Zubehöreinheit eine Lagerungseinheit, wie beispielsweise ein Lagerungskissen, zu korrekten und/oder bequemeren Lagerung und/oder Positionierung des Patienten auf dem Patiententisch umfassen.

Für eine medizinische Bildgebungsuntersuchung wird anschließend der Patient innerhalb eines Patientenaufnahmebereichs der medizinischen Bildgebungsvorrichtung gebracht. Innerhalb des Patientenaufnahmebereichs ist bevorzugt ein Field of View (FOV) und/oder ein Isozentrum der medizinischen Bildgebungsvorrichtung angeordnet. Das FOV umfasst bevorzugt einen Erfassungsbereich der medizinischen Bildgebungsvorrichtung, innerhalb dessen die Bedingungen für eine Erfassung von medizinischen Bilddaten vorliegen. Das Isozentrum der medizinischen Bildgebungsvorrichtung umfasst bevorzugt den Bereich und/oder Punkt innerhalb der medizinischen Bildgebungsvorrichtung, der die optimalen und/oder idealen Bedingungen für die Erfassung von medizinischen Bilddaten aufweist. Beispielsweise umfasst bei einer Magnetresonanzvorrichtung das Isozentrum den homogensten Magnetfeldbereich.

Die Gehäuseeinheit der medizinischen Bildgebungsvorrichtung ist an der Scannereinheit und/oder an der Patientenlagerungsvorrichtung angeordnet. Die Gehäuseeinheit umfasst zumindest eine funktionelle Gehäuseschale. Die zumindest eine funktionelle Gehäuseschale ist bevorzugt dazu ausgebildet, eine Erfassung einer möglichen Kontamination der Oberfläche der funktionellen Gehäuseschale und/oder eine Reinigung der Oberfläche der zumindest einen funktionellen Gehäuseschale zu unterstützen. Bevorzugt ist die funktionelle Gehäuseschale an Bereichen der Gehäuseeinheit angeordnet, die eine hohe Wahrscheinlichkeit einer Kontamination und/oder Verunreinigung während einer medizinischen Bildgebungsuntersuchung aufweisen. Oberflächen von Gehäuseschalen weisen eine hohe Wahrscheinlichkeit einer Kontamination auf, wenn sie in einem Kontaktbereich mit dem Patienten und/oder dem medizinischen Bedienpersonal angeordnet sind. Beispielsweise sind Oberflächen von Gehäuseschalen der Patientenlagerungsvorrichtung und/oder des Patientenaufnahmebereichs der medizinischen Bildgebungsvorrichtung und/oder ein Frontbereich der medizinischen Bildgebungsvorrichtung häufigen Kontakten mit dem Patienten und/oder dem medizinischen Bedienpersonal ausgesetzt.

Die zumindest eine funktionelle Gehäuseschale weist zumindest ein Sensorelement zu einer Erfassung eines Hygienestatus an der zumindest einen funktionellen Oberfläche auf. Der Hygienestatus kann dabei eine Kontamination und/oder Verunreinigung an der Oberfläche der funktionellen Gehäuseschale umfassen. Zudem kann der Hygienestatus auch eine Art einer Kontamination umfassen, wie beispielsweise eine Berührung oder ein Anhusten der Oberfläche usw. Weiterhin kann der Hygienestatus auch eine Information umfassen, dass keine Kontamination und/oder Verunreinigung der Oberfläche vorliegt. Zudem kann der Hygienestatus auch eine Information einer Reinigung der Oberfläche der funktionellen Gehäuseschale umfassen. Bevorzugt ist hierbei das zumindest eine Sensorelement in einer oberflächennahen Schicht der zumindest einen funktionellen Gehäuseschale angeordnet und/oder integriert für eine direkte Erfassung.

Eine Kontamination und/oder Verunreinigung der Oberfläche der zumindest einen funktionellen Gehäuseschale wird beispielsweise durch einen direkten Kontakt des Patienten und/oder des medizinischen Bedienpersonals mit der Oberfläche der funktionellen Gehäuseschale hervorgerufen. Zudem kann auch eine Kontamination und/oder Verunreinigung der Oberfläche der zumindest einen funktionellen Gehäuseschale durch ein Auftragen von Flüssigkeiten des Patienten, beispielsweise durch Husten oder Niesen, erfolgen.

Das zumindest eine Ausgabeelement ist zu einer visuellen Anzeige des Hygienestatus ausgebildet. Hierzu ist bevorzugt das zumindest eine Ausgabeelement derart in die zumindest eine funktionelle Gehäuseschale integriert, das die Anzeige des Hygienestatus für einen Benutzer direkt sichtbar ist. Beispielsweise kann ein Aufleuchten eines roten Lichts durch das zumindest eine Ausgabeelement dem Benutzer signalisieren, dass eine Kontamination und/oder Verunreinigung der Oberfläche der funktionellen Gehäuseschale vorliegt. Dagegen kann ein Aufleuchten eines grünen Lichts durch das zumindest eine Ausgabeelement dem Benutzer signalisieren, dass die Oberfläche der funktionellen Gehäuseschale frei von einer Kontamination und/oder Verunreinigung ist. Des Weiteren ist es auch denkbar, dass ein Aufleuchten eines blauen Lichts durch das zumindest einen Ausgabeelement dem Benutzer ein Andauern eines Reinigungsvorgangs signalisiert.

Das zumindest eine Reinigungselement der zumindest einen funktionellen Gehäuseschale ist zu einer Reinigung der Oberfläche der funktionellen Gehäuseschale ausgebildet. Bevorzugt ist hierbei das zumindest eine Reinigungselement in einer oberflächennahen Schicht der zumindest einen funktionellen Gehäuseschale angeordnet und/oder integriert. Das zumindest eine Reinigungselement kann derart ausgebildet sein, dass eine Reinigung durch ein Reinigungspersonal unterstützt wird, wie beispielsweise durch ein Bereitstellen einer Reinigungsflüssigkeit. Zudem kann das zumindest eine Reinigungselement auch zu einer automatischen Reinigung der Oberfläche der zumindest einen funktionellen Gehäuseschale ausgebildet sein.

Zudem kann die funktionelle Gehäuseschale auch mehr als ein Sensorelement und/oder mehr als ein Ausgabeelement und/oder mehr als ein Reinigungselement umfassen.

Die medizinische Bildgebungsvorrichtung weist bevorzugt eine Steuereinheit auf, die die von dem zumindest einen Sensorelement erfassten Daten auswertet und einen Hygienestatus für die Oberfläche der zumindest einen funktionellen Gehäuseschale ermittelt. Insbesondere kann auch das zumindest eine Ausgabeelement mittels der Steuereinheit anhand des ermittelten Hygienestatus angesteuert werden. Des Weiteren kann die Steuereinheit auch dazu ausgebildet sein, das zumindest eine Reinigungselement derart anzusteuern, dass eine automatische Reinigung der Oberfläche der zumindest einen funktionellen Gehäuseschale erfolgen kann. Weist die Gehäuseeinheit mehr als eine funktionelle Gehäuseschale auf, ist bevorzugt die Steuereinheit für eine Ansteuerung aller funktionellen Gehäuseschalen ausgebildet.

Die Erfindung weist den Vorteil auf, dass ein Benutzer, insbesondere ein medizinisches Bedienpersonal und/oder ein medizinisches Reinigungspersonal, bei einer Reinigung der medizinischen Bildgebungsvorrichtung nach einer medizinischen Bildgebungsuntersuchung vorteilhaft unterstützt werden kann. Insbesondere kann derart automatisch eine mögliche Kontamination von Oberflächen erfasst und für den Benutzer dargestellt werden. Durch visuelle Darstellung kann der Benutzer eine mögliche Kontamination und/oder Verschmutzung besonders schnell erfassen und einen möglichen Reinigungsaufwand und/oder einen Reinigungsworkflow effektiv abschätzen. Zudem kann der Benutzer auch eine vorteilhafte Unterstützung bei einer Reinigung der Oberflächen erhalten aufgrund des zumindest einen Reinigungselements. Dies ermöglicht auch, einen Reinigungsworkflow zeitsparend und effizient durchzuführen. Zudem wird durch die visuelle Darstellung auch eine Gefahr eines Übersehens einer Oberfläche bei einer Reinigung reduziert und damit auch eine Infektionsgefahr für einen Patienten verringert.

In einer vorteilhaften Weiterbildung der erfindungsgemäßen medizinischen Bildgebungsvorrichtung kann es vorgesehen sein, dass das zumindest eine Sensorelement einen Leitfähigkeitssensor, einen Feuchtesensor, einen Temperatursensor, einen optischen Sensor, einen chemischen und/oder elektrochemischen Sensor, einen kapazitiven Sensor, einen resistiven Sensor und/oder einen Gassensor umfasst. Besonders vorteilhaft kann dabei ein Gassensor auf Metalloxid-Basis zum Einsatz kommen, der sich durch eine sehr hohe Empfindlichkeit auszeichnet. Besonders vorteilhaft eignet sich auch ein flächiger, kapazitiver Sensor zur Erfassung eines Kontakts des Patienten und/oder des medizinischen Bedienpersonals mit der Oberfläche der funktionellen Gehäuseschale. Zudem kann das zumindest eine Sensorelement auch eine Kombination von mehreren, insbesondere unterschiedlichen Sensoren umfassen, so dass unterschiedliche Parameter zur Erfassung eines Hygienestatus erfasst werden können. In einer alternativen Ausgestaltung der Erfindung sind zudem weitere Ausgestaltungen des zumindest einen Sensorelements denkbar.

Diese Ausgestaltung der Erfindung ermöglicht eine einfache und schnelle Erfassung einer Kontamination und/oder Verunreinigung. Zudem kann derart auch ein Reinigungsvorgang vorteilhaft erfasst werden. Damit einhergehend kann auch ein Hygieneworkflow an die erfasste Kontamination und/oder Verunreinigung angepasst werden.

In einer vorteilhaften Weiterbildung der erfindungsgemäßen medizinischen Bildgebungsvorrichtung kann es vorgesehen sein, dass das zumindest eine Ausgabeelement eine OLED (organic light-emitting diode,), eine LED (Leuchtdiode oder ligthemitting diode), ein elektrochromes Ausgabeelement und/oder ein elektrolumineszierendes Ausgabeelement umfasst. Ein elektrolumineszierendes Ausgabeelement umfasst bevorzugt einen Festkörper, der aufgrund eines Anlegens eines elektrischen Feldes und/oder einer elektrischen Spannung eine elektromagnetische Strahlung, insbesondere in Form von Licht, aussendet und/oder emittiert. Ein elektrochromes Ausgabeelement umfasst bevorzugt ein Ausgabeelement, das seine optischen Eigenschaften aufgrund eines Anlegens eines elektrischen Feldes ändern kann. Diese Ausgestaltung der Erfindung weist den Vorteil auf, dass ein kompaktes und konstruktiv einfaches Ausgabeelement zur Anzeige des Hygienestatus für einen Benutzer bereitgestellt werden kann. Zudem kann eine besonders kostengünstiges Ausgabeelement für die Unterstützung einer Reinigung einer Oberfläche der funktionellen Gehäuseschale bereitgestellt werden.

In einer vorteilhaften Weiterbildung der erfindungsgemäßen medizinischen Bildgebungsvorrichtung kann es vorgesehen sein, dass das zumindest eine Reinigungselement ein Heizelement und/oder einen Fluidkanal umfasst. Bevorzugt umfasst das Heizelement einen Heizdraht und/oder einen Heizwiderstand, der dazu ausgebildet ist, elektrische Energie in thermische Energie umzuwandeln. Zudem kann das Heizelement auch eine Struktur aus Indiumzinnoxid (ITO) umfassen, wobei die Struktur aus ITO transparente Leiterbahnen umfassen kann für beispielsweise eine beheizbare transparente Schicht, insbesondere eine beheizbare transparente Oberflächenschicht und/oder Gehäuseschicht. Zudem kann die das Heizelement auch eine Struktur aus Zink-Aluminium-Oxid aufweisen, mittels der ebenfalls eine beheizbare transparente Schicht, insbesondere eine Schicht mit transparente Leiterbahnen realisiert werden kann. Weiterhin kann das Heizelement auch eine optisch transparente Schicht aus Kohlenstoffnanoröhren (CNT: carbon nanotubes) umfassen. Auch hier keine transparente Schicht mit Leiterbahnen realisiert werden. Beispielsweise kann hierbei eine Oberfläche und/oder eine äußere Gehäuseschicht der zumindest einen funktionellen Gehäuseschale auf ca. 200°C aufgeheizt werden. Bevorzugt erfolgt das Aufheizen innerhalb von wenigen Sekunden durch gezielte Hitzepulse.

Der Fluidkanal umfasst bevorzugt einen Kanal zur Verteilung eines Reinigungsmittels und/oder eines Desinfektionsmittels auf der Oberfläche der zumindest einen funktionellen Gehäuseschale. Dabei kann das Reinigungsmittel und/oder Desinfektionsmittels eine Flüssigkeit und/oder auch ein Gas, wie beispielsweise Ozon, Wasserstoffperoxid, Chloride, Peroxide usw., umfassen. Bevorzugt weist hierzu das zumindest eine Reinigungsmittel ein Pumpe zur Verteilung des Reinigungsmittels und/oder Desinfektionsmittels und/oder einen Behälter zur Aufnahme des Reinigungsmittels und/oder Desinfektionsmittels auf. Zudem kann der Fluidkanal auch zu einer Absaugung eines Reinigungsmittels, das bereits auf der Oberfläche der zumindest einen funktionellen Oberfläche verteilt ist, ausgebildet sein. Diese Ausgestaltung der Erfindung weist den Vorteil auf, dass eine einfache Reinigung der Oberfläche der zumindest einen funktionellen Gehäuseschale bereitgestellt werden kann. Insbesondere in Verbindung mit einer Steuereinheit kann eine automatische Reinigung der Oberfläche der zumindest einen funktionellen Gehäuseschale bereitgestellt werden und damit ein Reinigungspersonal vorteilhaft entlastet und/oder unterstützt werden.

In einer alternativen Ausgestaltung der Erfindung kann das zumindest eine Reinigungselement auch eine Strahlenquelle umfassen, die eine Reinigung und/oder Dekontamination der Oberfläche der zumindest einen funktionellen Gehäuseschale durch Bestahlen der Oberfläche erreicht. Die Strahlenquelle kann beispielsweise eine UV-C-Strahlenquelle umfassen. Des Weiteren kann die Strahlenquelle auch eine HINS-Elemente (High Intensity Narrow Spectrum) umfassen.

In einer vorteilhaften Weiterbildung der erfindungsgemäßen medizinischen Bildgebungsvorrichtung kann es vorgesehen sein, dass die zumindest eine funktionelle Gehäuseschale eine Schichtstruktur mit einer äußeren Gehäuseschicht aufweist, wobei das zumindest eine Reinigungselement und/oder das zumindest eine Sensorelement in der äußeren Gehäuseschicht angeordnet sind. Die Schichtstruktur der zumindest einen funktionellen Gehäuseschale weist zumindest zwei oder mehr Schichten auf. Die äußere Gehäuseschicht weist bevorzugt eine Oberflächenschicht auf, wobei die Oberflächenschicht diejenige Gehäuseschicht umfasst, die einer Kontamination und/oder Verunreinigung ausgesetzt ist. Zusätzlich kann es auch sein, dass das zumindest eine Ausgabeelement in der äußeren Gehäuseschicht der funktionellen Gehäuseschale angeordnet ist. Ist zumindest ein als Heizelement ausgebildetes Reinigungselement in der äußeren Gehäuseschicht angeordnet, ist bevorzugt das Heizelement derart ausgebildet, dass das Heizelement bei Kontakt mit Luft inert ist, wie beispielsweise ein Kupferdraht und/oder ein Heizelement aus Kohlenstoff.

Die Schichtstruktur der zumindest funktionellen Gehäuseschale weist bevorzugt eine Dicke von 50 µm bis zu 5 mm aufweisen. Bevorzugt umfasst die Schichtstruktur der zumindest funktionellen Gehäuseschale eine Dicke zwischen 100 µm und 500 µm. Besonders vorteilhaft umfasst die Schichtstruktur der zumindest funktionellen Gehäuseschale eine Dicke zwischen 100 µm und 200 µm.

Die Schichtstruktur kann dabei durch herkömmliche Verfahren, wie beispielsweise Laminieren und/oder Verkleben und/oder über ein physikalisches Gasphasenabscheidungsverfahren (PVD-Verfahren) und/oder ein chemisches Gasphasenabscheidungsverfahren (CVD-Verfahren) und/oder Photolithographie und/oder Ätzen usw. hergestellt werden. Besonders vorteilhaft kann die Schichtstruktur auch durch ein thermisches Spritzverfahren von Metallbahnen auf Kunststoff und/oder einen 3D-Druck hergestellt werden.

Diese Ausgestaltung der Erfindung weist den Vorteil auf, dass eine direkte Erfassung einer Verunreinigung und/oder auch direkte Bereitstellung einer Reinigung der Oberfläche erreicht werden kann. Zudem können auch Bauteile und/oder Elemente der funktionellen Gehäuseschale, die besonderen Schutz benötigen, in einer weiteren Gehäuseschicht angeordnet werden, wie beispielsweise in einer oberflächenfernen Gehäuseschicht.

In einer vorteilhaften Weiterbildung der erfindungsgemäßen medizinischen Bildgebungsvorrichtung kann es vorgesehen sein, dass die Schichtstruktur zumindest eine weitere Gehäuseschicht aufweist, in der das zumindest eine Ausgabeelement angeordnet ist. Derart kann das zumindest eine Ausgabeelement geschützt angeordnet werden. Umfasst beispielsweise das zumindest eine Reinigungselement ein Heizelement, kann es vorteilhaft sein, wenn das zumindest eine Ausgabeelement in einer von der das zumindest eine Reinigungselement umfassenden Gehäuseschicht unterschiedlichen und/oder getrennten Gehäuseschicht angeordnet ist.

In einer vorteilhaften Weiterbildung der erfindungsgemäßen medizinischen Bildgebungsvorrichtung kann es vorgesehen sein, dass die äußere Gehäuseschicht zumindest teilweise transparent ausgebildet ist. In diesem Zusammenhang soll unter einer zumindest teilweise transparent ausgebildeten Gehäuseschicht insbesondere verstanden werden, dass die Gehäuseschicht für sichtbares Licht, insbesondere für Licht mit einer Wellenlänge von 380 nm bis 750 nm, zumindest teilweise transparent ausgebildet ist. Dabei kann die äußere Gehäuseschicht Bereiche umfassen, die transparent ausgebildet sind und Bereiche umfassen, die nichttransparent ausgebildet sind. Bevorzugt sind diejenigen Bereich der äußeren Gehäuseschicht transparent ausgebildet, die ein Ausgabeelement der funktionellen Gehäuseschale überdecken. Diese Ausgestaltung der Erfindung weist den Vorteil auf, dass das zumindest eine Ausgabeelement geschützt unterhalb der äußeren Gehäuseschicht angeordnet werden kann und trotzdem für einen Benutzer gut sichtbar ist.

In einer alternativen Ausgestaltung kann die transparente Oberflächenschicht auch die gesamte äußere Schicht und/oder die gesamte Oberfläche der Gehäuseschale umfassen.

In einer vorteilhaften Weiterbildung der erfindungsgemäßen medizinischen Bildgebungsvorrichtung kann es vorgesehen sein, dass die äußere Gehäuseschicht eine transparente Trägerschicht umfasst und das zumindest eine Sensorelement und/oder das zumindest eine Reinigungselement in der transparenten Trägerschicht angeordnet ist. Bevorzugt ist die transparente Trägerschicht aus einem amorphen Material, insbesondere einem nicht kristallinen Material, gebildet, wie beispielsweise SiO₂. Zudem kann das auch das zumindest eine Reinigungselement ebenfalls zumindest teilweise transparent ausgebildet sein, wie beispielsweise ein Heizelement aus ITO und/oder Zink-Aluminium-Oxid und/oder Kohlenstoffnanoröhren usw. Derart kann vorteilhaft eine gute Sichtbarkeit von Bauteilen und/oder Elementen der Schichtstruktur, die unterhalb der äu-ßeren Gehäuseschicht angeordnet sind, wie beispielsweise eine Ausgabeelement, erreicht werden.

In einer vorteilhaften Weiterbildung der erfindungsgemäßen medizinischen Bildgebungsvorrichtung kann es vorgesehen sein, dass die Schichtstruktur ein Isolationselement aufweist, das zwischen dem zumindest einen Ausgabeelement und der äußeren Gehäuseschicht angeordnet ist. Bevorzugt umfasst das Isolationselement ein thermisches Isolationselement, das das zumindest eine Ausgabeelement vor einer Hitzeeinwirkung von beispielsweise einem Heizelement des zumindest einen Reinigungselements schützt. Zudem kann das Isolationselement weitere Bauteile der funktionellen Gehäuseschale vor einer Hitzeeinwirkung durch das Heizelement vorteilhaft schützen. Das Isolationselement kann dabei als Isolationsschicht ausgebildet sein, die insbesondere zwischen der äußeren Gehäuseschicht und der weiteren Gehäuseschicht mit dem zumindest einen Ausgabeelement angeordnet ist. Zudem kann das Isolationselement auch von der Trägerschicht umfasst sein, wobei hierbei die Trägerschicht bevorzugt gleichzeitig eine thermisch isolierende Wirkung auf die darunter angeordneten Bauteile und/oder Elemente der zumindest einen funktionellen Gehäuseschicht aufweist. Hierdurch kann ein vorteilhafter Schutz, insbesondere ein thermischer Schutz, von Bauteilen und/oder Bauelementen, insbesondere von dem zumindest einem Ausgabeelement, bei einer Reinigung durch Aufheizen einer Oberflächenschicht der zumindest einen funktionellen Gehäuseschale erreicht werden.

In einer vorteilhaften Weiterbildung der erfindungsgemäßen medizinischen Bildgebungsvorrichtung kann es vorgesehen sein, dass die Schichtstruktur eine Schutzschicht aufweist. Die Schutzschicht ist bevorzugt elektrisch leitfähig ausgebildet, um eine Erfassung einer Verschmutzung und/oder Kontamination durch das unterhalb der Schutzschicht angeordnete Sensorelement zu gewährleisten. Zudem ist die Schutzschicht ebenfalls transparent ausgebildet. Die Schutzschicht weist dabei eine Dicke zwischen 100 nm und 10 µm auf. Besonders vorteilhaft kann die Schutzschicht auch eine Dicke zwischen 200 nm und 800 nm aufweisen. Hierdurch können die oberflächennahen Schichten und/oder Bauteile, wie insbesondere das zumindest eine Sensorelement und/oder das zumindest eine Reinigungselement, vorteilhaft geschützt werden. Insbesondere können die Bauteile vor einer abrasiven Wischbewegung bei einer Reinigung geschützt werden. Gleichzeitig erlaubt zudem die Schutzschicht beispielsweise eine kapazitive Messung zur Erfassung eine Kontamination und/oder Verunreinigung.

In einer vorteilhaften Weiterbildung der erfindungsgemäßen medizinischen Bildgebungsvorrichtung kann es vorgesehen sein, dass die medizinische Bildgebungsvorrichtung eine Steuereinheit umfasst, die eine automatische Erfassung eines Hygienezustands an der zumindest einen funktionellen Gehäuseschale mittels des zumindest einen Sensorelements und eine automatische Ausgabe des Hygienestatus mittels des zumindest einen Ausgabeelements steuert.

Die erfindungsgemäße Steuereinheit umfasst zumindest ein Rechenmodul und/oder einen Prozessor, wobei die Steuereinheit zum Ausführen einer automatischen Steuerung des zumindest einen Sensorelements und des zumindest einen Ausgabeelements ausgebildet ist. So ist insbesondere die Steuereinheit dazu ausgebildet, computerlesbare Instruktionen auszuführen für eine automatischen Steuerung des zumindest einen Sensorelements und des zumindest einen Ausgabeelements. Insbesondere umfasst die Steuereinheit eine Speichereinheit, wobei auf der Speichereinheit computerlesbare Informationen gespeichert sind, wobei die Steuereinheit dazu ausgebildet ist, die computerlesbaren Informationen von der Speichereinheit zu laden und die computerlesbaren Informationen auszuführen.

Die Komponenten der Steuereinheit können zum überwiegenden Teil in Form von Softwarekomponenten ausgebildet sein. Grundsätzlich können diese Komponenten aber auch zum Teil, insbesondere wenn es um besonders schnelle Berechnungen geht, in Form von softwareunterstützten Hardwarekomponenten, beispielsweise FPGAs oder dergleichen, realisiert sein. Ebenso können die benötigten Schnittstellen, beispielsweise wenn es nur um eine Übernahme von Daten aus anderen Softwarekomponenten geht, als Softwareschnittstellen ausgebildet sein. Sie können aber auch als hardwaremäßig aufgebaute Schnittstellen ausgebildet sein, die durch geeignete Software angesteuert werden. Selbstverständlich ist es auch denkbar, dass mehrere der genannten Komponenten in Form einer einzelnen Softwarekomponente bzw. softwareunterstützter Hardwarekomponente zusammengefasst realisiert sind.

Die Steuereinheit kann dabei innerhalb der zumindest einen funktionellen Gehäuseschale integriert sein. Zudem ist es auch denkbar, dass die Steuereinheit außerhalb der zumindest einen funktionellen Gehäuseschale angeordnet ist, beispielsweise wenn die Steuereinheit zu einer Steuerung von mehreren funktionellen Gehäuseschalen ausgebildet ist. Vorzugsweise ist die Steuereinheit auch zu einer Auswertung der erfassten Sensordaten ausgebildet.

Diese Ausgestaltung der Erfindung weist den Vorteil auf, dass eine schnelle automatische Erfassung und Anzeige eines Hygienestatus für einen Benutzer bereitgestellt werden kann. Vorteilhafterweise ist die Steuereinheit derart ausgebildet, dass eine Kontamination und/oder eine Verunreinigung während einer medizinischen Bildgebungsuntersuchung gespeichert wird und erst nach der medizinischen Bildgebungsuntersuchung für den Benutzer angezeigt und/oder dargestellt wird.

In einer vorteilhaften Weiterbildung der erfindungsgemäßen medizinischen Bildgebungsvorrichtung kann es vorgesehen sein, dass die Steuereinheit eine automatische Reinigung der zumindest einen funktionellen Gehäuseschale, insbesondere der Oberfläche der funktionellen Gehäuseschale, mittels des zumindest einen Reinigungselements steuert. Beispielsweise kann die Steuereinheit das zumindest eine Reinigungselement derart ansteuern das bei einer Verunreinigung und/oder Kontamination der zumindest einen funktionellen Gehäuseschale die Reinigung beginnt, sobald die medizinische Bildgebungsuntersuchung beendet ist. In einer alternativen Ausgestaltung kann die Reinigung auch erst beginnen, sobald ein Benutzer eine Eingabe tätigt, die als Triggerereignis zum Auslösen eines Reinigungsvorgangs von der Steuereinheit erfasst wird.

Derart kann vorteilhaft eine automatische Reinigung der zumindest einen funktionellen Gehäuseschale erreicht werden und damit ein Reinigungspersonal entlastet werden. Zudem kann derart sichergestellt werden, dass ein versehentliches Vergessen einer Reinigung, beispielsweise aufgrund von Zeitdruck des Reinigungspersonals, verhindert wird. Weiterhin kann auch eine Infektionsgefahr für die Patienten reduziert werden.

Des Weiteren geht die Erfindung aus von einem Verfahren zu einem Reinigen einer Oberfläche einer funktionellen Gehäuseschale einer medizinischen Bildgebungsvorrichtung, wobei das Verfahren die folgenden Schritte umfasst:
- Erfassen eines Hygienestatus der Oberfläche der funktionellen Gehäuseschale mittels zumindest eines Sensorelements,
- Ausgabe des Hygienestatus an einen Benutzer mittels zumindest eines Ausgabeelements, und
- sofern ein Hygienestatus vorliegt, der eine Kontamination und/oder Verunreinigung der Oberfläche umfasst, Reinigen der Oberfläche mittels des zumindest einen Reinigungselements.

Vorzugsweise wird das Verfahren mithilfe einer Steuereinheit der funktionellen Gehäuseschale durchgeführt, wobei die Steuereinheit die einzelnen Einheiten und/oder Bauteile, insbesondere das zumindest eine Sensorelement, das zumindest eine Ausgabeelement und/oder das zumindest eine Reinigungselement zur Ausführung des Verfahren ansteuert. So erfolgt die Ausgabe eines Hygienestatus, der eine Kontamination und/oder Verunreinigung umfasst, mittels des zumindest einen Ausgabeelements nicht unmittelbar nach der Erfassung und/oder Ermittlung des Hygienestatus, sondern bevorzugt erst nach einem Beenden der medizinischen Bildgebungsuntersuchung, um eine Verwirrung des Patienten während der medizinischen Bildgebungsuntersuchung zu verhindern. Zudem beginnt auch die Reinigung der Oberfläche der funktionellen Gehäuseschale erst nach dem Beenden der medizinischen Bildgebungsuntersuchung. Zudem kann die Reinigung auch erst nach einer Benutzereingabe ausgelöst werden.

Hierdurch kann vorteilhaft erreicht werden, dass ein Benutzer, insbesondere ein medizinisches Bedienpersonal und/oder ein medizinisches Reinigungspersonal, bei einer Reinigung der medizinischen Bildgebungsvorrichtung nach einer medizinischen Bildgebungsuntersuchung vorteilhaft unterstützt wird. Insbesondere kann derart automatisch eine mögliche Kontamination von Oberflächen erfasst werden. Durch visuelle Darstellung kann der Benutzer eine mögliche Kontamination und/oder Verschmutzung besonders schnell erfassen und einen möglichen Reinigungsaufwand und/oder einen Reinigungsworkflow effektiv abschätzen. Zudem kann der Benutzer auch eine vorteilhafte Unterstützung bei einer Reinigung der Oberflächen erhalten aufgrund des zumindest einen Reinigungselements. Dies ermöglicht auch, einen Reinigungsworkflow zeitsparend und effizient durchzuführen. Zudem wird durch die visuelle Darstellung auch eine Gefahr eines Übersehens einer Oberfläche bei einer Reinigung reduziert und damit auch eine Infektionsgefahr für einen Patienten verringert.

Die Vorteile des erfindungsgemäßen Verfahrens zu einem Reinigen einer Oberfläche einer funktionellen Gehäuseschale einer medizinischen Bildgebungsvorrichtung entsprechen im Wesentlichen den Vorteilen der erfindungsgemäßen medizinischen Bildgebungsvorrichtung, welche vorab im Detail ausgeführt sind. Hierbei erwähnte Merkmale, Vorteile oder alternative Ausführungsformen können ebenso auf die anderen beanspruchten Gegenstände übertragen werden und umgekehrt.

In einer vorteilhaften Weiterbildung des erfindungsgemäßen Verfahren kann es vorgesehen sein, dass eine Steuerung des Sensorelements, des Ausgabeelements und/oder des Reinigungselements mittels einer Steuereinheit erfolgt. Hierdurch kann eine vorteilhafte Automatisierung des Verfahren erreicht werden und damit auch ein Benutzer bei der Reinigung entlastet werden.

Weitere Vorteile, Merkmale und Einzelheiten der Erfindung ergeben sich aus den im Folgenden beschriebenen Ausführungsbeispielen sowie anhand der Zeichnungen.

Es zeigen:
- Fig. 1: eine erfindungsgemäße medizinische Bildgebungsvorrichtung mit einer funktionellen Gehäuseschale in einer schematischen Darstellung,
- Fig. 2: ein erstes Ausführungsbeispiel einer funktionellen Gehäuseschale in einer Schnittdarstellung,
- Fig. 3: ein zweites Ausführungsbeispiel einer funktionellen Gehäuseschale in einer Schnittdarstellung,
- Fig. 4: ein drittes Ausführungsbeispiel einer funktionellen Gehäuseschale in einer Schnittdarstellung,
- Fig. 5: ein viertes Ausführungsbeispiel einer funktionellen Gehäuseschale in einer Draufsicht,
- Fig. 6: das vierte Ausführungsbeispiel der funktionellen Gehäuseschale in einer Schnittdarstellung,
- Fig. 7: ein Verfahren zu einem Reinigen einer Oberfläche einer funktionellen Gehäuseschale einer medizinischen Bildgebungsvorrichtung.

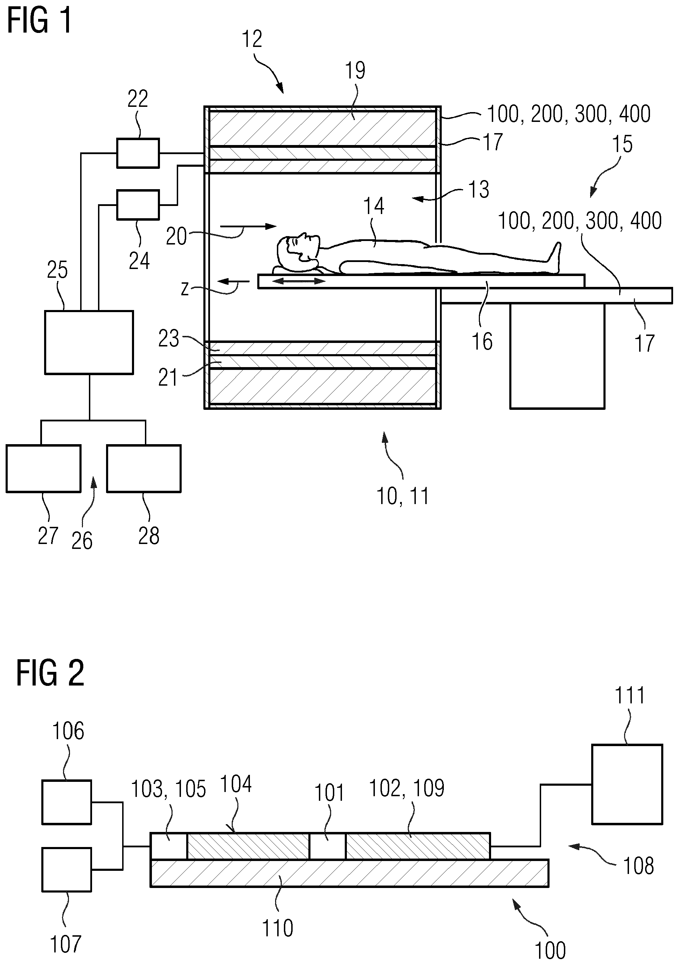

In Fig. 1 ist eine medizinische Bildgebungsvorrichtung 10 schematisch dargestellt. Die medizinische Bildgebungsvorrichtung 10 ist im vorliegenden Ausführungsbeispiel von einer Magnetresonanzvorrichtung 11 gebildet, wobei beispielhaft die vorliegende Erfindung anhand einer Magnetresonanzvorrichtung 11 erläutert wird. Die vorliegende Erfindung ist jedoch nicht auf Ausgestaltung der medizinischen Bildgebungsvorrichtung 10 auf eine Magnetresonanzvorrichtung 11 beschränkt und weitere Ausgestaltungen der medizinischen Bildgebungsvorrichtung 10 sind jederzeit denkbar.

Die Magnetresonanzvorrichtung 11 umfasst eine von einer Magneteinheit gebildeten Scannereinheit 12. Zudem weist die Magnetresonanzvorrichtung 11 einen Patientenaufnahmebereich 13 auf zu einer Aufnahme eines Patienten 14. Der Patientenaufnahmebereich 13 im vorliegenden Ausführungsbeispiel ist zylinderförmig ausgebildet und in einer Umfangsrichtung von der Scannereinheit 12, insbesondere von der Magneteinheit, zylinderförmig umgeben. Grundsätzlich ist jedoch eine davon abweichende Ausbildung des Patientenaufnahmebereichs 13 jederzeit denkbar. Der Patient 14 kann mittels einer Patientenlagerungsvorrichtung 15 der Magnetresonanzvorrichtung 11 in den Patientenaufnahmebereich 13 geschoben und/oder gefahren werden. Die Patientenlagerungsvorrichtung 15 weist hierzu einen innerhalb des Patientenaufnahmebereichs 13 bewegbar ausgestalteten Patiententisch 16 auf. Insbesondere ist hierbei der Patiententisch 16 in Richtung einer Längserstreckung des Patientenaufnahmebereichs 13 und/oder in z-Richtung bewegbar gelagert.

Des Weiteren weist die medizinische Bildgebungsvorrichtung 10, insbesondere die Magnetresonanzvorrichtung 11, eine Gehäuseeinheit 17 auf. Die Gehäuseeinheit 17 ist dabei an der Scannereinheit 12 und auch an der Patientenlagerungsvorrichtung 15 angeordnet. Die Gehäuseeinheit 17 weist zumindest eine funktionelle Gehäuseschale 100, 200, 300, 400 auf. Im vorliegenden Ausführungsbeispiel weist die Gehäuseeinheit 17 mehrere funktionelle Gehäuseschalen 100, 200, 300, 400 auf, die bevorzugt an Bereichen der Scannereinheit 12 und der Patientenlagerungsvorrichtung 13 angeordnet sind, die eine hohe Kontaminationswahrscheinlichkeit und/oder Kontaktwahrscheinlichkeit mit einem Patienten 14 und/oder dem medizinischen Bedienpersonal aufweisen. Insbesondere umfassen hierbei eine Frontseite der Scannereinheit 12 und/oder eine um den Patientenaufnahmebereich 13 angeordnete Umhausung funktionelle Gehäuseschalen 100, 200, 300, 400 auf. Zudem kann auch die Patientenlagerungsvorrichtung 15 im Bereich des Patiententischs 16 funktionelle Gehäuseschalen 100, 200, 300, 400 aufweisen.

Die Scannereinheit 12, insbesondere die Magneteinheit, umfasst einen supraleitenden Grundmagneten 19 zu einem Erzeugen eines starken und insbesondere konstanten Grundmagnetfelds 20. Weiterhin weist die Scannereinheit 12, insbesondere die Magneteinheit, eine Gradientenspuleneinheit 21 zu einer Erzeugung von Magnetfeldgradienten auf, die für eine Ortskodierung während einer Bildgebung verwendet werden. Die Gradientenspuleneinheit 21 wird mittels einer Gradientensteuereinheit 22 der Magnetresonanzvorrichtung 11 gesteuert. Die Scannereinheit 12, insbesondere die Magneteinheit, umfasst weiterhin eine Hochfrequenzantenneneinheit 23 zu einer Anregung einer Polarisation, die sich in dem von dem Grundmagneten 19 erzeugten Grundmagnetfeld 20 einstellt. Die Hochfrequenzantenneneinheit 23 wird von einer Hochfrequenzantennensteuereinheit 24 der Magnetresonanzvorrichtung 11 gesteuert und strahlt hochfrequente Magnetresonanzsequenzen in den Patientenaufnahmebereich 13 der Magnetresonanzvorrichtung 11 ein.

Zu einer Steuerung des Grundmagneten 19, der Gradientensteuereinheit 22 und zur Steuerung der Hochfrequenzantennensteuereinheit 24 weist die Magnetresonanzvorrichtung 11 eine Systemsteuereinheit 25 auf. Die Systemsteuereinheit 25 steuert zentral die Magnetresonanzvorrichtung 11, wie beispielsweise das Durchführen einer vorbestimmten bildgebenden Gradientenechosequenz. Zudem umfasst die Systemsteuereinheit 25 eine nicht näher dargestellte Auswerteeinheit zu einer Auswertung von medizinischen Bilddaten, die während der Magnetresonanzuntersuchung erfasst werden.

Des Weiteren umfasst die medizinische Bildgebungsvorrichtung 10, insbesondere die Magnetresonanzvorrichtung 11, eine Benutzerschnittstelle 26, die mit der Systemsteuereinheit 25 verbunden ist. Steuerinformationen wie beispielsweise Bildgebungsparameter, sowie rekonstruierte Magnetresonanzbilder können auf einer Anzeigeeinheit 27, beispielsweise auf zumindest einem Monitor, der Benutzerschnittstelle 26 für ein medizinisches Bedienpersonal angezeigt werden. Weiterhin weist die Benutzerschnittstelle 26 eine Eingabeeinheit 28 auf, mittels der Informationen und/oder Parameter während eines Messvorgangs von dem medizinischen Bedienpersonal eingegeben werden können.

Die dargestellte medizinische Bildgebungsvorrichtung 10, insbesondere die Magnetresonanzvorrichtung 11, kann selbstverständlich weitere Komponenten umfassen, die medizinische Bildgebungsvorrichtungen 10, insbesondere die Magnetresonanzvorrichtungen 11, gewöhnlich aufweisen. Eine allgemeine Funktionsweise einer medizinischen Bildgebungsvorrichtung 10, insbesondere der Magnetresonanzvorrichtung 11, ist zudem dem Fachmann bekannt, so dass auf eine detaillierte Beschreibung der weiteren Komponenten verzichtet wird.

Im Folgenden werden die funktionellen Gehäuseschalen 100, 200, 300, 400 näher beschrieben, wobei die Beschreibung beispielhaft anhand einer einzigen Gehäuseschale 100, 200, 300, 400 erfolgt.

In Fig. 2 ist ein erstes Ausführungsbeispiel einer funktionellen Gehäuseschale 100 dargestellt. Die funktionelle Gehäuseschale 100 weist ein Sensorelement 101, ein Ausgabeelement 102 und ein Reinigungselement 103 auf. Die funktionelle Gehäuseschale 100 kann dabei auch mehr als ein Sensorelement 101 und/oder mehr als ein Ausgabeelement 102 und/oder mehr als ein Reinigungselement 103 aufweisen.

Das Sensorelement 101 ist zu einer Erfassung eines Hygienestatus an der funktionellen Gehäuseschale 100, insbesondere an der Oberfläche 104 der funktionellen Gehäuseschale 100, ausgebildet. Das Sensorelement 101 kann dabei einen Leitfähigkeitssensor, einen Feuchtesensor, einen Temperatursensor, einen optischen Sensor, einen chemischen Sensor, einen elektrochemischen Sensor, einen kapazitiven Sensor, einen resistiven Sensor und/oder einen Gassensor usw. umfassen. Das Ausgabeelement 102 ist zu einer visuellen Ausgabe des Hygienestatus ausgebildet und weist eine OLED, eine LED, ein elektrochromes Ausgabeelement und/oder ein elektrolumineszierendes Ausgabeelement usw. auf.

Das Reinigungselement 103 ist zu einer Reinigung der Oberfläche 104 der funktionellen Gehäuseschale 100 ausgebildet und umfasst im vorliegenden Ausführungsbeispiel eine Kanalstruktur mit einem Fluidkanal 105. In dem Fluidkanal 105 ist ein Reinigungsmittel geführt, dass durch eine Öffnung des Fluidkanals 105 an der Oberfläche 104 der funktionellen Gehäuseschale 100 austreten kann und sich dort verteilt. Hierzu weist das Reinigungselement 103 eine Pumpe 106, beispielsweise eine Mikropumpe, und ein Reinigungsmittelreservoir 107 auf, die in Fig 2 nur schematisch angedeutet sind. Die Pumpe 106 und das Reinigungsmittelreservoir 107 können dabei auch von der funktionellen Gehäuseschale 100 umfasst sein oder auch zentral von der Gehäuseeinheit 17.

Die funktionelle Gehäuseschale 100 umfasst des Weiteren eine Schichtstruktur 108 mit einer äußeren Gehäuseschicht 109. In dieser äußeren Gehäuseschicht 109 ist das Sensorelement 101, das Ausgabeelement 102 und das Reinigungselement 103 angeordnet. Zudem umfasst die funktionelle Gehäuseschale 100 eine weitere Gehäuseschicht 110, die eine Trägerschicht umfasst. Die funktionelle Gehäuseschale 100 mit der Schichtstruktur 108 weist dabei eine Dicke zwischen von 50 µm bis zu 5 mm auf.

Zu einer Steuerung des Sensorelements 101, insbesondere zu einer Auswertung der erfassten Sensordaten und zur Ermittlung des Hygienestatus, weist die Gehäuseeinheit 17 eine Steuereinheit 111 auf. Die Steuereinheit 111 kann dabei von der funktionellen Gehäuseschale 100 umfasst sein oder auch separat zu dieser ausgebildet sein oder auch zentral von der Gehäuseeinheit 18 umfasst sein. Die Steuereinheit 111 ist hierbei dazu ausgebildet, anhand der erfassten Daten des Sensorelements 101 den Hygienestatus der Oberfläche 104 der funktionellen Gehäuseschale 100 zu ermitteln. Der Hygienestatus kann dabei Kontamination und/oder eine Verunreinigung der Oberfläche 104 umfassen. Zudem kann ein Hygienestatus auch eine Information umfassen, dass keine Kontamination und/oder Verunreinigung der Oberfläche 104 vorliegt. Zudem kann der Hygienestatus auch einen Reinigungsprozess umfassen.

Mittels der Steuereinheit 111 wird auch das Ausgabeelement 102 angesteuert. Vorzugsweise ist die Steuereinheit 111 dazu ausgebildet, eine automatische Ausgabe des erfassten und/oder ermittelten Hygienestatus mittels des zumindest einen Ausgabeelements 102 zu steuern. Beispielsweise kann bei einem Hygienestatus, der eine Kontamination und/oder eine Verunreinigung der Oberfläche 104 der funktionellen Gehäuseschale 100 umfasst, durch Ausgabe eines roten Lichts mittels des Ausgabeelements 102 angezeigt werden. Liegt dagegen ein Hygienestatus vor, der keine Kontamination und/oder Verunreinigung der Oberfläche 104 der funktionellen Gehäuseschale 100 umfasst, kann dies beispielsweise durch Ausgabe eines grünen Lichts mittels des Ausgabeelements 102 angezeigt werden. Zudem kann ein Hygienestatus, der einen Reinigungsprozess der Oberfläche 104 der funktionellen Gehäuseschale 100 umfasst, durch Ausgabe eines blauen Lichts mittels des Ausgabeelements 102 angezeigt werden. Dabei kann das Ausgabeelement 102 derart von der Steuereinheit 111 gesteuert werden, dass eine Anzeige eines Hygienestatus erst nach der medizinischen Bildgebungsuntersuchung erfolgt, um beispielsweise einen Patienten 14 während der medizinische Bildgebungsuntersuchung nicht zu beunruhigen.

Zudem ist die Steuereinheit 11 auch dazu ausgebildet, eine Reinigung mittels des Reinigungselements 103 zu steuern. Vorzugsweise wird hierbei die Reinigung von der Steuereinheit 111 auf die medizinische Bildgebungsuntersuchung abgestimmt und erfolgt hierbei erst nach der medizinischen Bildgebungsuntersuchung. Zudem kann es auch sein, dass die Steuereinheit 111 eine Reinigung der Oberfläche 104 der funktionellen Gehäuseschale 100 erst startet, wenn von einem Benutzer eine Starteingabe für einen Reinigungsvorgang eingegeben wird. Während einer Reinigung wird dabei ein Reinigungsmittel durch den Fluidkanal 105 gepumpt und kann an definierten Stellen an die Oberfläche 104 der funktionellen Gehäuseschale 100 austreten und sich auf der Oberfläche 104 verteilen.

Für eine Ansteuerung des Sensorelements 101, des Ausgabeelements 102 und des Reinigungselements 103 weist die Steuereinheit 111 eine entsprechende Steuersoftware und/oder Computerprogramme auf, die bei einem Ausführen in einem Prozessor der Steuereinheit 111 die Steuerung des Sensorelements 101, des Ausgabeelements 102 und des Reinigungselements 103 ausführen.

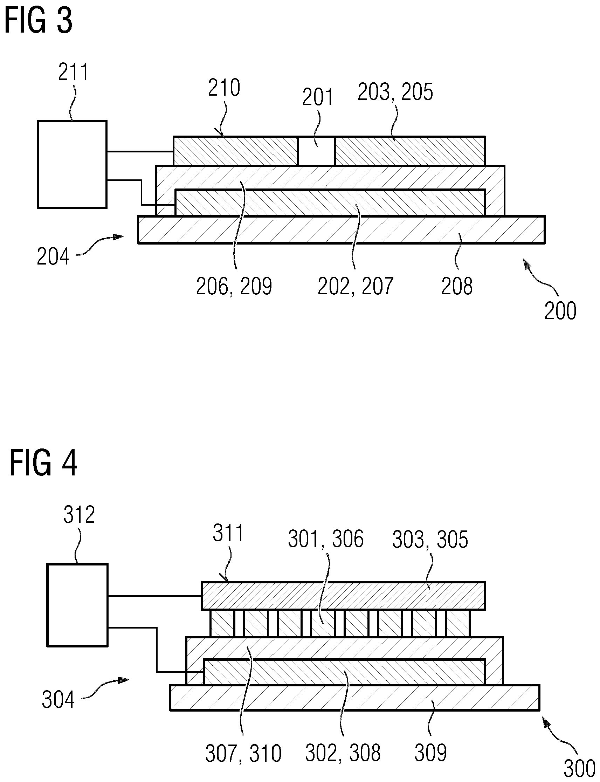

In Fig. 3 ist ein alternatives Ausführungsbeispiel der funktionellen Gehäuseschale 200 dargestellt. Im Wesentlichen gleichbleibende Bauteile, Merkmale und Funktionen sind grundsätzlich mit den gleichen Bezugszeichen beziffert. Die nachfolgende Beschreibung beschränkt sich im Wesentlichen auf die Unterschiede zu dem Ausführungsbeispiel in der Fig. 2, wobei bezüglich gleichbleibender Bauteile, Merkmale und Funktionen auf die Beschreibung des Ausführungsbeispiels in der Fig. 2 verwiesen wird.

In Fig. 3 ist eine zweites Ausführungsbeispiel der funktionellen Gehäuseschale 200 dargestellt. Die funktionelle Gehäuseschale 200 weist hierbei ein Sensorelement 201, ein Ausgabeelement 202 und ein Reinigungselement 203 auf. Die funktionelle Gehäuseschale 200 kann dabei auch mehr als ein Sensorelement 201 und/oder mehr als ein Ausgabeelement 202 und/oder mehr als ein Reinigungselement 203 aufweisen. Hierbei ist das Sensorelement 201 wie in der Beschreibung zu Fig. 2 ausgebildet. Zudem ist auch das Ausgabeelement 202 wie in der Beschreibung zu Fig. 2 ausgebildet. Das Reinigungselement 203 umfasst im vorliegenden Ausführungsbeispiel ein Heizelement, wie beispielsweise einen Heizwiderstand und/oder ein Heizelement, dass bei Kontakt mit Luft inert ist, wie insbesondere ein Kupferdraht und/oder ein Heizelement aus Kohlenstoff.

Die funktionelle Gehäuseschale 200 weist hierbei eine Schichtstruktur 204 mit einer äußeren Gehäuseschicht 205 auf. Die äußere Gehäuseschicht 205 umfasst hierbei eine Oberflächenschicht der funktionellen Gehäuseschale 200. In dieser äußeren Gehäuseschicht 205 ist das Reinigungselement 203 und das Sensorelement 201 angeordnet. Zudem weist die Schichtstruktur 204 der funktionellen Gehäuseschale 200 weitere Gehäuseschichten 206, 207, 208 auf, wobei eine der weiteren Gehäuseschichten 207 das Ausgabeelement 202 umfasst. Zwischen der äußeren Gehäuseschicht 204 und der das Ausgabeelement 202 umfassenden Gehäuseschicht 207 ist eine Gehäuseschicht 206 mit einem Isolationselement 209 und/oder eine Isolationsschicht angeordnet, um das Ausgabeelement 202 vor einer Überhitzung zu schützen. Bevorzugt ist das Isolationselement 209 und/oder die Isolationsschicht thermisch isolierend ausgebildet.

Um eine Ausgabe des Ausgabeelements 202 für einen Benutzer sichtbar zu machen, ist das Isolationselement 209 und die äu-ßere Gehäuseschicht 205 zumindest teilweise transparent ausgebildet. Hierzu ist beispielsweise die Gehäuseschicht 206 mit dem Isolationselement 209 und/oder der Isolationsschicht aus Glas und/oder einem transparenten Kunststoff, insbesondere einen hitzebeständigen transparenten Kunststoff, wie beispielsweise ein Polyimid, gebildet. In der äußeren Gehäuseschicht 205 weist hierzu beispielsweise das Heizelement, insbesondere der Heizwiderstand, eine Struktur aus Indiumzinnoxid (ITO) auf, wobei die Struktur aus ITO transparente Leiterbahnen umfassen kann für beispielsweise eine beheizbare Schicht. Beispielsweise wird hierbei die äußere Gehäuseschicht 205 und/oder eine Oberfläche 210 der funktionellen Gehäuseschale 200 auf ca. 200°C aufgeheizt. Bevorzugt kann das Aufheizen innerhalb von wenigen Sekunden durch gezielte Hitzepulse erfolgen. Zudem kann die äußere Gehäuseschicht 205 auch eine Trägerstruktur und/oder ein Trägermaterial umfassen, in der das Heizelement angeordnet ist, wobei die Trägerstruktur und/oder das Trägermaterial bevorzugt transparent ausgebildet ist, wie beispielsweise aus Siliziumdioxid (SiO₂).

Zudem umfasst die Schichtstruktur 204 der funktionelle Gehäuseschale 200 eine weitere Gehäuseschicht 208, die eine Trägerschicht umfasst. Bevorzugt ist die Trägerschicht die oberflächenfernste Gehäuseschicht 208 der Schichtstruktur 204.

Die funktionelle Gehäuseschale 200 weist des Weiteren ebenfalls eine Steuereinheit 211, wobei eine Funktionsweise der Steuereinheit 211 den Ausführungen zu Fig. 2 entspricht. Insbesondere erfolgt eine Wirkungsweise und/oder Ansteuerung des Sensorelements 201, des Ausgabeelements 202 den Ausführungen zu Fig. 2. Auch eine Ansteuerung des Reinigungselements 203 erfolgt gemäß den Ausführungen zu Fig. 2, wobei im vorliegenden Ausführungsbeispiel bei einer Aktivierung des Reinigungselements 203 durch die Steuereinheit 211 ein kurzzeitiges Aufheizen der Oberfläche 210 der funktionellen Gehäuseschale 200 auf ca. 200°C erfolgt.

In Fig. 4 ist ein alternatives Ausführungsbeispiel der funktionellen Gehäuseschale 300 dargestellt. Im Wesentlichen gleichbleibende Bauteile, Merkmale und Funktionen sind grundsätzlich mit den gleichen Bezugszeichen beziffert. Die nachfolgende Beschreibung beschränkt sich im Wesentlichen auf die Unterschiede zu dem Ausführungsbeispiel in den Fig. 2 und 3, wobei bezüglich gleichbleibender Bauteile, Merkmale und Funktionen auf die Beschreibung des Ausführungsbeispiels in den Fig. 2 und 3 verwiesen wird.

In Fig. 4 ist eine drittes Ausführungsbeispiel der funktionellen Gehäuseschale 300 dargestellt. Die funktionelle Gehäuseschale 300 weist hierbei ein Sensorelement 301, ein Ausgabeelement 302 und ein Reinigungselement 303 auf. Die funktionelle Gehäuseschale 300 kann dabei auch mehr als ein Sensorelement 301 und/oder mehr als ein Ausgabeelement 302 und/oder mehr als ein Reinigungselement 303 aufweisen.

Auch hier weist die funktionelle Gehäuseschale 300 eine Schichtstruktur 304 mit einer äußeren Gehäuseschicht 305 und weiteren Gehäuseschichten 306, 307, 308 309 auf. Die äußere Gehäuseschicht 305 umfasst in diesem Ausführungsbeispiel ausschließlich das Reinigungselement 303, das entsprechend zu den Ausführungen zu Fig. 3 ausgebildet ist. Zudem weist eine der weiteren Gehäuseschichten 307 ein Isolationselement 310 und/oder eine Isolationsschicht auf, die entsprechend zu den Ausführungen zu Fig. 3 ausgebildet ist. Eine weitere Gehäuseschicht 308 umfasst das Ausgabeelement 302, wie dies bereits in den Ausführungen zu Fig. 3 beschrieben ist. Zudem umfasst die Schichtstruktur 304 der funktionelle Gehäuseschale 300 eine weitere Gehäuseschicht 309, die eine Trägerschicht umfasst. Bevorzugt ist die Trägerschicht die oberflächenfernste Gehäuseschicht 309 der Schichtstruktur 304.

Zwischen der äußeren Gehäuseschicht 305 und der weiteren Gehäuseschicht 307 mit dem Isolationselement 310 und/oder der Isolationsschicht ist eine weitere Gehäuseschicht 306 der funktionellen Gehäuseschale 300 angeordnet, die das Sensorelement 301 umfasst. Das Sensorelement 301 umfasst im vorliegenden Ausführungsbeispiel einen taktilen Sensor, wie beispielsweise einen kapazitiven Sensor zur Erfassung eines Kontakts des Patienten 14 und/oder des Benutzers mit der Oberfläche 311 der funktionellen Gehäuseschale 300.

Die funktionelle Gehäuseschale 300 weist des Weiteren ebenfalls eine Steuereinheit 312, wobei eine Funktionsweise der Steuereinheit den Ausführungen zu Fig. 2 entspricht. Insbesondere erfolgt eine Wirkungsweise und/oder Ansteuerung des Sensorelements 301, des Ausgabeelements 302 und des Reinigungselements 303 den Ausführungen zu den Fig. 2 und 3.

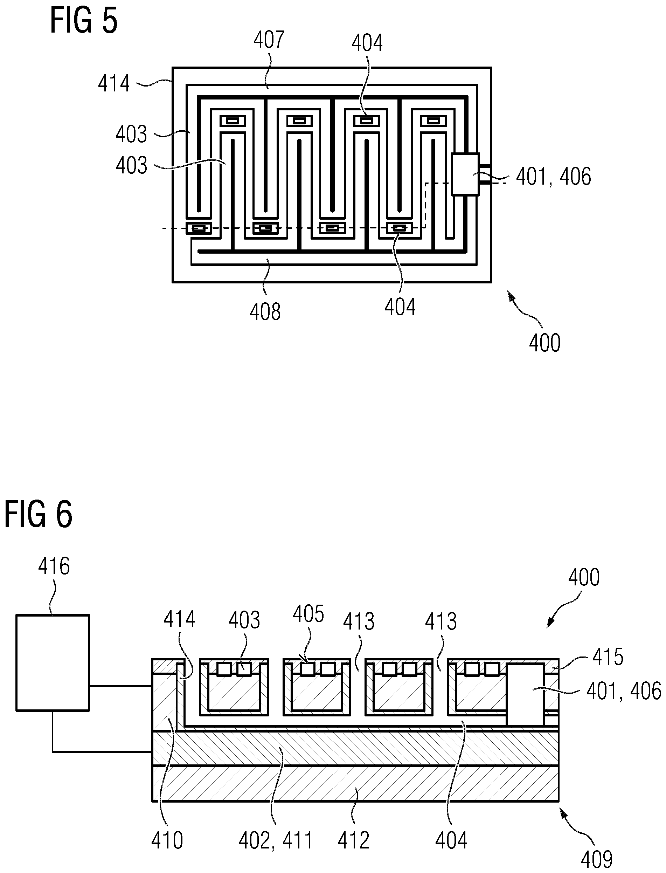

In den Fig. 5 und 6 ist ein alternatives Ausführungsbeispiel der funktionellen Gehäuseschale 400 dargestellt. Im Wesentlichen gleichbleibende Bauteile, Merkmale und Funktionen sind grundsätzlich mit den gleichen Bezugszeichen beziffert. Die nachfolgende Beschreibung beschränkt sich im Wesentlichen auf die Unterschiede zu dem Ausführungsbeispiel in den Fig. 2 bis 4, wobei bezüglich gleichbleibender Bauteile, Merkmale und Funktionen auf die Beschreibung des Ausführungsbeispiels in den Fig. 2 bis 4 verwiesen wird.

In den Fig. 5 und 6 ist eine viertes Ausführungsbeispiel der funktionellen Gehäuseschale 400 dargestellt. Die funktionelle Gehäuseschale 400 weist hierbei ein Sensorelement 401, ein Ausgabeelement 402 und zwei Reinigungselemente 403, 404 auf. Die funktionelle Gehäuseschale 400 kann dabei auch mehr als ein Sensorelement 401 und/oder mehr als ein Ausgabeelement 402 aufweisen. Ein erstes Reinigungselement 403 umfasst ein Heizelement mit zwei Heizkreisen 407, 408. Mittels der beiden Heizkreise 407, 408 kann die Oberfläche 405 der funktionellen Gehäuseschale 400 kurzzeitig aufgeheizt werden, beispielsweise auf ca. 200 °C. Das erste Reinigungselement 403 weist im vorliegenden Ausführungsbeispiel zudem ein Steuerelement 406 auf, das die beiden Heizkreise 407, 408 miteinander verbindet. Zudem können mittels des Steuerelements 406 die Heizkreise 407, 408 kapazitiv ausgelesen werden und damit das erste Reinigungselement 403, insbesondere die beiden Heizkreise 407, 408 zusammen mit dem Steuerelement 406, als Sensorelement 401 der funktionellen Gehäuseschale 400 fungieren. Derart können Kontakte einer Person mit der Oberfläche 405 der funktionellen Gehäuseschale 400 und/oder Verunreinigungen von beispielsweise Flüssigkeiten erfasst werden. Ein zweites Reinigungselement 404 umfasst einen Fluidkanal und ist von der Wirkungsweise sinngemäß zu dem Reinigungselement 203, insbesondere dem Fluidkanal, in Fig. 2 ausgebildet. Dabei weist das zweite Reinigungselement 404 ebenfalls eine nicht näher dargestellte Pumpe und ein nicht näher dargestelltes Reinigungsreservoir auf.

Auch hier weist die funktionelle Gehäuseschale 400 eine Schichtstruktur 409 mit einer äußeren Gehäuseschicht 410 und weiteren Gehäuseschichten 411, 412 auf. In der äußeren Gehäuseschicht 410 sind die beiden Reinigungselemente 403, 404 und damit auch das Sensorelement 401 angeordnet. Wie in Fig. 5 und 6 zu sehen ist, sind in Bereichen zwischen den beiden Heizkreisen 407, 408 regelmäßige Öffnungen 413 angeordnet, durch die eine Reinigungsflüssigkeit auf der Oberfläche 405 der funktionellen Gehäuseschale 400 verteilt werden kann. Zudem umfasst die äußere Gehäuseschicht 410 eine transparente Trägerschicht 414, wobei die beiden Reinigungselemente 403, 404 und damit auch das Sensorelement 401 innerhalb der Trägerschicht 414 angeordnet sind. Bevorzugt sind hierzu auch die beiden Reinigungselemente 403, 404 zumindest teilweise transparent angeordnet. Eine einer weiteren Gehäuseschicht 411 zugewandte Seite der Trägerschicht 414 dient zugleich als thermisches Isolationselement für eine weitere, unterhalb der äußeren Gehäuseschicht 410 angeordneten Gehäuseschicht 411. In dieser weiteren Gehäuseschicht 411 ist das Ausgabeelement 402 angeordnet, das entsprechend den Ausführungen zu der Beschreibung zu Fig. 2 ausgebildet ist.

Des Weiteren weist die funktionelle Gehäuseschale400, insbesondere die Schichtstruktur 409 der funktionellen Gehäuseschale 400, eine Schutzschicht 415 auf, auf der Oberfläche 405 der funktionellen Gehäuseschale 400 angeordnet ist. Die Schutzschicht 415 hat die Aufgabe, die Oberfläche 405 der funktionellen Gehäuseschale 400 zu schützen, beispielweise bei abrasiven Wischbewegungen während einer Reinigung der Oberfläche 405 der funktionellen Gehäuseschale 400. Die Schutzschicht 415 weist hierbei eine Dicke zwischen 100 nm und 10 µm auf. Besonders vorteilhaft kann die Schutzschicht 415 auch eine Dicke zwischen 200 nm und 800 nm aufweisen. Die Schutzschicht 415 ist bevorzugt elektrisch leitfähig ausgebildet, um eine Erfassung einer Verschmutzung und/oder Kontamination durch das unterhalb der Schutzschicht 415 angeordnete Sensorelement 401 zu gewährleisten.

Zudem umfasst die Schichtstruktur 409 der funktionelle Gehäuseschale 400 eine weitere Gehäuseschicht 412, die eine Trägerschicht umfasst. Bevorzugt ist die Trägerschicht die oberflächenfernste Gehäuseschicht 412 der Schichtstruktur 409. Das Ausgabeelement 402 ist dabei zwischen der Trägerschicht und der äußeren Gehäuseschicht 410 angeordnet.

Die funktionelle Gehäuseeinheit 400 weist des Weiteren eine Steuereinheit 416 auf, wobei eine Ansteuerung des Sensorelements 401, des Ausgabeelement 402 und der Reinigungselemente 403, 404 entsprechend der Beschreibung in Fig. 2 und 3 erfolgt.

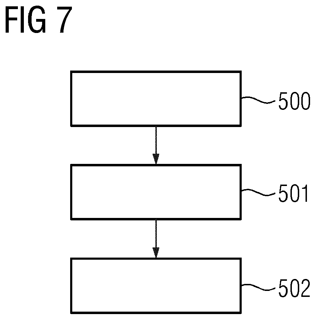

In Fig. 7 ist ein Verfahren zu einem Reinigen einer Oberfläche 104, 210, 311, 405 einer funktionellen Gehäuseschale 100, 200, 300, 400 einer medizinischen Bildgebungsvorrichtung 10 dargestellt. In einem ersten Verfahrensschritt 500 erfolgt ein Erfassen eines Hygienestatus der Oberfläche 104, 210, 311, 405 der funktionellen Gehäuseschale 100, 200, 300, 400 mittel zumindest eines Sensorelements 101, 201, 301, 401 der funktionellen Gehäuseschale 100, 200, 300, 400. Hierbei werden die von dem Sensorelement 101, 201, 301, 401 erfassten Daten von der Steuereinheit 111, 211, 312, 416 hinsichtlich eines Hygienestatus ausgewertet. Die Erfassung mittels des Sensorelements 101, 201, 301, 401 erfolgt dabei bereits während einer Vorbereitung des Patienten 14 für die medizinische Bildgebungsuntersuchung. Zudem erfolgt die Erfassung auch während der medizinischen Bildgebungsuntersuchung.

In einem weiteren Verfahrensschritt 501 erfolgt eine Ausgabe des Hygienestatus an einen Benutzer mittels eines Ausgabeelements 102, 202, 302, 402 der funktionellen Gehäuseschale 100, 200, 300, 400. Die Ausgabe des Hygienestatus erfolgt dabei gesteuert von der Steuereinheit 111, 211, 312, 416. Bevorzugt erfolgt die Ausgabe mittels des Ausgabeelements 102, 202, 302, 402 nach einem Beenden der medizinischen Bildgebungsuntersuchung. Beispielsweise kann ein Aufleuchten eines roten Lichts durch das Ausgabeelement 102, 202, 302, 402 dem Benutzer signalisieren, dass eine Kontamination und/oder Verunreinigung der Oberfläche 104, 210, 311, 405 der funktionellen Gehäuseschale 100, 200, 300, 400 vorliegt. Dagegen kann ein Aufleuchten eines grünen Lichts durch das Ausgabeelement 102, 202, 302, 402 dem Benutzer signalisieren, dass die Oberfläche 104, 210, 311, 405 der funktionellen Gehäuseschale 100, 200, 300, 400 frei von einer Kontamination und/oder Verunreinigung ist. Des Weiteren ist es auch denkbar, dass ein Aufleuchten eines blauen Lichts durch das Ausgabeelement 102, 202, 302, 402 dem Benutzer ein Andauern eines Reinigungsvorgangs signalisiert.

Sofern ein Hygienestatus vorliegt, der eine Kontamination und/oder Verunreinigung der Oberfläche 104, 210, 311, 405 der funktionellen Gehäuseschale 100, 200, 300, 400 umfasst, erfolgt ein Reinigen der Oberfläche 104, 210, 311, 405 mittels zumindest eines Reinigungselements 103, 203, 303, 403, 404 der funktionellen Gehäuseschale 100, 200, 300, 400. Die Reinigung der Oberfläche erfolgt dabei gesteuert mittels der Steuereinheit 111, 211, 312, 416. Bevorzugt erfolgt die Reinigung nach einem Beenden der medizinischen Bildgebungsuntersuchung. Zudem kann es sein, dass vor einem Beginn eines Reinigungsvorgangs eine Starteingabe mittels der Eingabeeinheit der medizinischen Bildgebungsvorrichtung tätigen muss.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Medizinische Bildgebungsvorrichtung mit einer Scannereinheit, einer Patientenlagerungsvorrichtung und einer Gehäuseeinheit, die an der Scannereinheit und/oder an der Patientenlagerungsvorrichtung angeordnet ist, wobei die Gehäuseeinheit zumindest eine funktionelle Gehäuseschale umfasst,
wobei
die zumindest eine funktionelle Gehäuseschale zumindest ein Sensorelement zur Erfassung eines Hygienestatus an der zumindest einen funktionellen Gehäuseschale, zumindest ein Ausgabeelement, das zu einer visuellen Anzeige des Hygienestatus ausgebildet ist, und zumindest ein Reinigungselement zur Reinigung einer Oberfläche der zumindest einen funktionellen Gehäuseschale umfasst.

2. Medizinische Bildgebungsvorrichtung nach Anspruch 1, wobei
das zumindest eine Sensorelement einen Leitfähigkeitssensor, einen Feuchtesensor, einen Temperatursensor, einen optischen Sensor, einen chemischen Sensor, einen elektrochemischen Sensor, einen kapazitiven Sensor, einen resistiven Sensor und/oder einen Gassensor umfasst.

3. Medizinische Bildgebungsvorrichtung nach einem der vorhergehenden Ansprüche,
wobei
das zumindest eine Ausgabeelement eine OLED, eine LED, ein elektrochromes Ausgabeelement und/oder ein elektrolumineszierendes Ausgabeelement umfasst.

4. Medizinische Bildgebungsvorrichtung nach einem der vorhergehenden Ansprüche,
wobei
das zumindest eine Reinigungselement ein Heizelement und/oder einen Fluidkanal umfasst.

5. Medizinische Bildgebungsvorrichtung nach einem der vorhergehenden Ansprüche,
wobei
die zumindest eine funktionelle Gehäuseschale eine Schichtstruktur mit einer äußeren Gehäuseschicht aufweist, wobei das zumindest eine Reinigungselement und/oder das zumindest eine Sensorelement in der äußeren Gehäuseschicht angeordnet sind.

6. Medizinische Bildgebungsvorrichtung nach Anspruch 5,
wobei
die Schichtstruktur zumindest eine weitere Gehäuseschicht aufweist, in der das zumindest eine Ausgabeelement angeordnet ist.

7. Medizinische Bildgebungsvorrichtung nach einem der Ansprüche 5 bis 6,
wobei
die äußere Gehäuseschicht zumindest teilweise transparent ausgebildet ist.

8. Medizinische Bildgebungsvorrichtung nach einem der Ansprüche 5 bis 7,
wobei
die äußere Gehäuseschicht eine transparente Trägerschicht umfasst und das zumindest eine Sensorelement und/oder das zumindest eine Reinigungselement in der transparente Trägerschicht angeordnet ist.

9. Medizinische Bildgebungsvorrichtung nach einem der Ansprüche 5 bis 8,
wobei
die Schichtstruktur ein Isolationselement aufweist, das zwischen dem zumindest einen Ausgabeelement und der äußeren Gehäuseschicht angeordnet ist.

10. Medizinische Bildgebungsvorrichtung nach einem der Ansprüche 5 bis 9,
wobei
die Schichtstruktur eine Schutzschicht aufweist.

11. Medizinische Bildgebungsvorrichtung nach einem der vorhergehenden Ansprüche,
weiterhin umfassend
eine Steuereinheit, die eine automatische Erfassung eines Hygienezustands an der zumindest einen funktionellen Gehäuseschale mittels des zumindest einen Sensorelements und eine automatische Ausgabe des Hygienestatus mittels des zumindest einen Ausgabeelements steuert.

12. Medizinische Bildgebungsvorrichtung nach Anspruch 11,
wobei
die Steuereinheit eine automatische Reinigung der zumindest einen funktionellen Gehäuseschale mittels des zumindest einen Reinigungselements steuert.

13. Verfahren zu einem Reinigen einer Oberfläche einer funktionellen Gehäuseschale einer medizinischen Bildgebungsvorrichtung, die nach einem der Ansprüche 1 bis 12 ausgebildet ist, wobei das Verfahren die folgenden Schritte umfasst:
- Erfassen eines Hygienestatus der Oberfläche der funktionellen Gehäuseschale mittels des zumindest einen Sensorelements,
- Ausgabe des Hygienestatus an einen Benutzer mittels des zumindest einen Ausgabeelements, und
- sofern ein Hygienestatus vorliegt, der eine Kontamination und/oder Verunreinigung der Oberfläche umfasst, Reinigen der Oberfläche mittels des zumindest einen Reinigungselements.

14. Verfahren nach Anspruch 13,
wobei
eine Steuerung des Sensorelements, des Ausgabeelements und/oder des Reinigungselements mittels einer Steuereinheit erfolgt.

## Claims

1. Medical imaging apparatus with a scanner unit, a patient support apparatus and a housing unit, which is arranged on the scanner unit and/or on the patient support apparatus, wherein the housing unit comprises at least one functional housing shell,
wherein the at least one functional housing shell comprises at least one sensor element for detection of a hygiene status on the at least one functional housing shell, at least one output element, which is embodied for a visual display of the hygiene status, and at least one cleaning element for cleaning a surface of the at least one functional housing shell.

2. Medical imaging apparatus according to claim 1,
wherein the at least one sensor element comprises a conductivity sensor, a moisture sensor, a temperature sensor, an optical sensor, a chemical sensor, an electrochemical sensor, a capacitive sensor, a resistive sensor and/or gas sensor.

3. Medical imaging apparatus according to one of the preceding claims,
wherein the at least one output element comprises an OLED, an LED, an electrochromic output element and/or an electroluminescent output element.

4. Medical imaging apparatus according to one of the preceding claims,
wherein the at least one cleaning element comprises a heating element and/or a fluid channel.

5. Medical imaging apparatus according to one of the preceding claims,
wherein the at least one functional housing shell has a layer structure with an outer housing layer, wherein the at least one cleaning element and/or the at least one sensor element is arranged in the outer housing layer.

6. Medical imaging apparatus according to claim 5,
wherein the layer structure has at least one further housing layer, in which the at least one output element is arranged.

7. Medical imaging apparatus according to one of claims 5 to 6,
wherein the outer housing layer is embodied at least partly transparent.

8. Medical imaging apparatus according to one of claims 5 to 7,
wherein the outer housing layer comprises a transparent carrier layer and the at least one sensor element and/or the at least one cleaning element is arranged in the transparent carrier layer.

9. Medical imaging apparatus according to one of claims 5 to 8,
wherein the layer structure has an insulation element, which is arranged between the at least one output element and the outer housing layer.

10. Medical imaging apparatus according to one of claims 5 to 9,
wherein the layer structure has a protective layer.

11. Medical imaging apparatus according to one of the preceding claims,
further comprising a control unit, which controls an automatic detection of a hygiene status on the at least one functional housing shell by means of the at least one sensor element and an automatic output of the hygiene status by means of the at least one output element.

12. Medical imaging apparatus according to claim 11,
wherein the control unit controls an automatic cleaning of the at least one functional housing shell by means of the at least one cleaning element.

13. Method for cleaning a surface of a functional housing shell of a medical imaging apparatus, which is embodied according to one of claims 1 to 12, wherein the method comprises the following steps:
- detection of a hygiene status of the surface of the functional housing shell by means of the at least one sensor element,
- output of the hygiene status to a user by means of the at least one output element, and
- provided a hygiene status is present that comprises a contamination and/or soiling of the surface, cleaning of the surface by means of the at least one cleaning element.

14. Method according to claim 13,
wherein the sensor element, the output element and/or the cleaning element are controlled by means of a control unit.

## Revendications

1. Installation d'imagerie médicale ayant une unité de scanner, une couchette de patient et une unité d'enveloppe, qui est disposée sur l'unité de scanner et/ou sur la couchette de patient, dans laquelle l'unité d'enveloppe comprend au moins une coque d'enveloppe fonctionnelle,
dans laquelle la au moins une coque d'enveloppe fonctionnelle comprend au moins un élément capteur pour la détection d'un statut d'hygiène sur la au moins une coque d'enveloppe fonctionnelle, au moins un élément d'émission, qui est constitué pour une indication visuelle du statut d'hygiène, et au moins un élément de nettoyage pour le nettoyage d'une surface de la au moins une coque d'enveloppe fonctionnelle.

2. Installation d'imagerie médicale suivant la revendication 1,
dans laquelle le au moins un élément capteur comprend un capteur de conductivité, un capteur d'humidité, un capteur de température, un capteur optique, un capteur chimique, un capteur électrochimique, un capteur capacitif, un capteur résistif et/ou un capteur de gaz.

3. Installation d'imagerie médicale suivant l'une des revendications précédentes,
dans laquelle le au moins un élément d'émission comprend une OLED, une LED, un élément d'émission électrochrome et/ou un élément d'émission électroluminescent.

4. Installation d'imagerie médicale suivant l'une des revendications précédentes,
dans laquelle le au moins un élément de nettoyage comprend un élément de chauffage et/ou un conduit pour du fluide.

5. Installation d'imagerie médicale suivant l'une des revendications précédentes,
dans laquelle la au moins une coque d'enveloppe fonctionnelle a une structure en couche ayant une couche extérieure d'enveloppe, dans laquelle le au moins un élément de nettoyage et/ou le au moins un élément capteur sont disposés sur la couche extérieure de l'enveloppe.

6. Installation d'imagerie médicale suivant la revendication 5,
dans laquelle la structure en couche à moins une autre couche d'enveloppe, dans laquelle est disposée le au moins un élément d'émission.

7. Installation d'imagerie médicale suivant l'une des revendications 5 à 6,
dans laquelle la couche extérieure de l'enveloppe est transparente au moins en partie.

8. Installation d'imagerie médicale suivant l'une des revendications 5 à 7,
dans laquelle la couche extérieure de l'enveloppe comprend une couche support transparente et le au moins un élément capteur et/ou le au moins un élément de nettoyage est disposé dans la couche de support transparente.

9. Installation d'imagerie médicale suivant l'une des revendications 5 à 8,
dans laquelle la structure en couche a un élément isolant, qui est disposé entre le au moins un élément d'émission et la couche extérieure de l'enveloppe.

10. Installation d'imagerie médicale suivant l'une des revendications 5 à 9,
dans laquelle la structure en couche a une couche de protection.

11. Installation d'imagerie médicale suivant l'une des revendications précédentes,
comprenant en outre une unité de commande, qui commande une détection automatique d'un état d'hygiène sur la au moins une coque d'enveloppe fonctionnelle au moyen du au moins un élément capteur et une émission automatique de statut d'hygiène au moyen du au moins un élément d'émission.

12. Installation d'imagerie médicale suivant la revendication 11,
dans laquelle l'unité de commande commande un nettoyage automatique de la au moins une coque d'enveloppe fonctionnelle au moyen du au moins un élément de nettoyage.

13. Procédé de nettoyage d'une surface d'une coque d'enveloppe fonctionnelle d'une installation d'imagerie médicale, qui est constituée suivant l'une des revendications 1 à 12, dans lequel le procédé comprend les stades suivants :
- détection d'un statut d'hygiène de la surface de la coque d'enveloppe fonctionnelle au moyen du au moins un élément capteur,
- envoi du statut d'hygiène à un utilisateur au moyen du au moins un élément d'émission,
- dans la mesure où il y a un statut d'hygiène, qui comprend une contamination et/ou une pollution de la surface, nettoyage de la surface au moyen du au moins un élément de nettoyage.

14. Procédé suivant la revendication 13,
dans lequel une commande de l'élément capteur, de l'élément d'émission et/ou de l'élément de nettoyage s'effectue au moyen d'une unité de commande.
